# FASCICULE DE BREVET EUROPEEN

(11) **EP 0 345 152 B1**
(45) Date de publication et mention de la délivrance du brevet: **04.09.1996**
(21) Numéro de dépôt: 89401483.6
(22) Date de dépôt: 31.05.1989
(51) Int. Cl.: C12N 15/00, C12P 21/00

(54) **Baculovirus modifié, son procédé de préparation et son application en tant que vecteur d'expression de gènes**
Modifiziertes Baculovirus,Herstellung und Verwendung als Expressionsvektor
Modified Baculovirus, preparation and use as an expression vector

(30) Priorité: 31.05.1988 FR 8807207
(43) Date de publication de la demande: 06.12.1989
(73) Titulaire: INSTITUT NATIONAL DE LA RECHERCHE AGRONOMIQUE (INRA), F-75341 Paris Cédéx 07 (FR); CENTRE NATIONAL DE LA RECHERCHE SCIENTIFIQUE (CNRS), 75794 Paris Cédex 16 (FR)
(72) Inventeur: Devauchelle, Gérard, F-30380 Saint-Christol-les-Ales (FR); Cerutti née Duonor, Martine, F-30380 Saint-Christol-les-Ales (FR); Croizier, Guy, F-30380 Saint-Christol-les-Ales (FR); Croizier, née Robin, Liliane, F-30380 Saint-Christol-les-Ales (FR)
(74) Mandataire: Ores, Irène

(56) Documents cités:
- EP-A- 0 040 466
- EP-A- 0 074 808
- EP-A- 0 127 839
- EP-A- 0 228 036
- WO-A-86/02380
- WO-A-88/07082
- US-A- 4 419 446
- CHEMICAL ABSTRACTS vo. 107, no. 25, 21 decembre 1987, page 227 abrege no. 230511z, Columbus, Ohio, USA; P. GONNET et al.: "Recombination of the P10 polypeptide gene to obtain a baculovirus expressing the neomycin resistance gene in insect cells" & C. R. Acad. Sci. Ser. 3, vol. 305, no. 5, 1987, pages 111-114
- AGRICULTURAL & BIOLOGICAL CHEMISTRY vol. 51, no. 6, juin 1987, pages 1573-1580, Tokyo, Japan; T. HORIUCHI et al.: "High-level Expression of the Human-alpha-interferon Gene through the Use of an Improved Baculovirus Vector in the Silkworm. Bombyx mori" page 1574, colonne 2; page 1576; colonne 2

## Description

La présente invention est relative à un baculovirus modifié possédant un seul site de coupure derrière le promoteur du polypeptide majeur de l'inclusion virale de baculovirus, la polyédrine, et/ou de la protéine P 10, à un procédé pour l'obtention d'un tel baculovirus modifié et à son application en tant que vecteur d'expression de gènes.

Le génie génétique utilise des systèmes de vecteurs viraux eucaryotes pour introduire par transduction des ADN exogènes dans des cellules animales ou végétales. Les principaux virus eucaryotes qui sont susceptibles de servir de vecteurs de transduction d'ADN dans des cellules de mammifères (SV40 et polyome, par exemple) et dans des cellules végétales (virus de la mosaïque du chou-fleur, par exemple) ne peuvent recevoir que de l'ADN exogène ("étranger") dont la longueur est limitée, en raison de la morphologie de la structure de leur nucléocapside. Du fait que la taille des génomes est considérablement augmentée par la présence des séquences introduites, il s'est avéré absolument nécessaire de pouvoir disposer de vecteurs susceptibles de "loger" plus d'ADN exogène, "étranger", d'autant plus que le génie génétique, en se développant, tend à chercher à insérer dans une cellule-hôte plus d'un gène étranger, dans le but, par exemple, d'obtenir une expression coordonnée et, si possible, une activité coordonnée des produits des gènes étrangers. Le vecteur viral idéal devrait, en outre, permettre l'introduction d'un long segment d'ADN étranger dans des cellules, à une fréquence élevée et permettre la conversion de la biosynthèse de toutes les protéines cellulaires en l'expression du ou des gène(s) étranger(s). Un virus paraissant remplir toutes ces conditions est le baculovirus suivant : virus de la polyédrose nucléaire, Autographa californica (AcNPV) qui, selon Loïs K. MILLER (Chapitre 14 "A virus vector for genetic engineering in invertebrates" du Manuel "GENETIC ENGINEERING IN PLANT SCIENCES" (1981) N.J. PANOPOULOS, ED., PRAEGER PUBL. NEW-YORK, pages 203-223), constitue un excellent vecteur de propagation et d'expression de nombreux gènes étrangers dans un environnement eucaryote. Selon cette Etude, l'AcNPV présente deux formes, virus non inclus, NOV, et virus inclus, OV, qui jouent des rôles différents dans le processus d'infection par baculovirus, et les gènes qui codent pour la polyédrine ne sont pas nécessaires et pourraient être éliminés et remplacés par de l'ADN étranger introduit dans la séquence nucléotidique codant pour la polyédrine, pour procurer un système vecteur capable de donner un taux élevé d'expression des gènes étrangers. Il est également indiqué dans cette Etude que la polyédrine, qui est la protéine cristalline qui forme la matrice du corps d'inclusion de l'AcNPV,et dont le poids moléculaire est de 30KD, est synthétisée en très grandes quantités par l'OV, sans qu'il y ait amplification du gène de la polyédrine dans le processus de replication, ce qui laisse à penser que le promoteur de la synthèse de l'ARNm de la polyédrine est exceptionnellement puissant et qu'il pourrait être utilisé pour obtenir des taux élevés d'expression d'un gène étranger. L'Auteur indique, en outre, qu'il a été établi précédemment que le génome, lui-même identifié, de l'AcNPV, code pour le gène de la polyédrine. De plus, un certain nombre de variants génotypiques de l'AcNPV ont été observés et des variations des sites d'endonucléases de restriction pour plusieurs des variants qui se forment naturellement, ont été cartographiées.

Dans cette Etude, L. MILLER rappelle que le génome (ADN) de l'AcNPV est infectieux dans des cultures cellulaires et que l'aptitude de ce génome à transfecter des cultures cellulaires est de nature à permettre de lier de l'ADN étranger à l'ADN viral, d'insérer l'ADN recombinant dans des cellules en culture par un processus de transfection et d'obtenir un virus contenant les séquences d'ADN additionnelles et que cette aptitude est également de nature à permettre la manipulation in vitro de l'ADN de l'AcNPV ou d'un recombinant et sa réinsertion aisée dans des cellules, la grande taille du génome de l'AcNPV - qui, selon les estimations, aurait un poids moléculaire de 82-88 millions de daltons ou un poids moléculaire de 92 millions de daltons - constituant un avantage dans la perspective de l'introduction de grands segments d'ADN étranger dans les cellules-hôtes. Il est indiqué dans cette Etude qu'en raison des grandes quantités de polyédrine produites dans les cellules infectées, il y a avantage à remplacer le gène de la polyédrine par de l'ADN étranger, en utilisant, pour l'expression, le promoteur de la polyédrine.

Un Article de K.N. POTTER et L. K. MILLER paru dans ANIMAL VIRUS GENETICS, 6, GENETIC MUTATIONS OF A BACULOVIRUS, ACADEMIC PRESS (1980), pages 71 - 80, décrit la méthode du "marker rescue" (récupération des marqueurs) d'établissement de la carte génétique de mutants ts d'AcNPV sur la base de la carte de restriction des sites de plusieurs endonucléases de restriction, laquelle méthode consiste à recombiner in vivo un génome d'ADN mutant avec un fragment de restriction d'ADN d'AcNPV sauvage.

Un certain nombre de Publications font application des données exposées par L.MILLER dans son Etude. C'est ainsi que les Demandes de Brevet européen N° 0127 839 au nom de THE TEXAS A&M UNIVERSITY SYSTEM (avec MM. G.E. SMITH et D. SUMMERS mentionnés comme Inventeurs), N° 0228 036 au nom de MICROGENESYS (avec M. M. COCHRAN mentionné comme Inventeur) et N° 0260 090 aux noms de D.H.L. BISHOP et C.Y. KANG, qui sont déposants et Inventeurs, ont toutes trois pour objet de produire un vecteur d'expression d'un baculovirus recombinant, qui est ensuite utilisé pour infecter une cellule d'insecte-hôte sensible. Selon ces procédés, on isole d'abord d'un baculovirus approprié, tel qu'Autographa californica (AcMNPV), un fragment d'ADN qui comprend le promoteur de la polyédrine et des séquences d'ADN codant pour la protéine de polyédrine. Selon la première de ces trois Publications, le fragment isolé est inséré dans un vecteur de clonage tel que le plasmide pUC 8, pour former un vecteur de transfert constitué par un véhicule de clonage (le plasmide) qui contient obligatoirement le promoteur de polyédrine (mais pas obligatoirement des séquences d'ADN codant pour la polyédrine) et un site disponible pour cloner un gène sélectionné qui se trouvera sous le contrôle transcriptionnel dudit promoteur ; puis un vecteur de transfert recombinant est formé par insertion dans le site de clonage disponible susdit, d'un gène sélectionné, en utilisant les techniques d'ADN recombinant, et enfin, à partir de ce vecteur de transfert recombinant, on forme un vecteur d'expression recombinant en incorporant, par transfection, dans l'ADN de baculovirus, un fragment du vecteur de transfert recombinant. Le vecteur d'expression de baculovirus recombinant formé, est capable d'exprimer le gène sélectionné inséré dans le vecteur de transfert recombinant. La deuxième de ces trois Publications vise à produire un polypeptide tel que l'antigène de surface du virus de l'hépatite B, dans une cellule-hôte infectée par un virus, en isolant d'un baculovirus (virus capable d'infecter une cellule-hôte d'insecte), un premier segment d'ADN incluant un promoteur viral tel que le promoteur de polyédrine, puis en isolant d'une source appropriée, un second segment d'ADN qui contient la séquence qui code pour le polypeptide et en combinant ces deux segments d'ADN pour former un brin continu d'un troisième segment d'ADN qui contient de l'ADN vecteur dans lequel le second segment d'ADN est adjacent au promoteur du premier segment d'ADN et contient des signaux de fin de transcription ; on forme ensuite un vecteur recombinant en recombinant le troisième segment susdit avec de l'ADN génomique de baculovirus, après quoi le vecteur recombinant est mis en contact avec des cellules-hôtes d'insecte dans des conditions qui provoquent l'incorporation du segment de vecteur recombinant dans lesdites cellules-hôtes, pour les infecter ; elles sont ensuite mises en culture pour isoler des cellules ou des surnageants de culture, l'antigène de surface du virus de l'hépatite B. La troisième de ces Publications (Demande de Brevet européen 0260090) a pour objet un procédé de production d'un polypeptide qui comprend au moins une fraction antigénique de la protéine de l'antigène de surface du virus de l'hépatite B (HBsAg) ou de la protéine Pre-S2, par infection d'insectes ou de cellules d'insectes sensibles, par un vecteur d'expression constitué par un baculovirus recombinant comportant un segment d'ADN qui code pour le polypeptide recherché (HBsAg), sous le contrôle d'expression d'un promoteur de polyédrine, le baculovirus recombinant étant lui-même obtenu par cotransfection de cultures de cellules d'insectes par de l'ADN de baculovirus infectieux et des plasmides vecteurs de transfert de baculovirus contenant des gènes représentant les antigènes du virus de l'hépatite B, qui sont placés à l'emplacement des séquences initiales 5′ codantes du gène de polyédrine de baculovirus, mais sous le contrôle du promoteur de polyédrine.

Ces trois Publications ont en commun la nécessité d'utiliser pour obtenir le vecteur d'expression, un vecteur de transfert qui est un véhicule qui est chargé d'un gène étranger (gène de l'interféron β dans la Demande de Brevet européen 0127 839 ; gène de l'HBsAg dans les deux autres Demandes de Brevets européens 0228 036 et 0260 090). Lorsque le vecteur de transfert est chargé, il est nécessaire de procéder au transfert du gène étranger dans le virus, ce qui est réalisé, conformément à ces Demandes de Brevets, par cotransfection, après quoi on recherche et isole les virus recombinants qui constituent les vecteurs d'expression, que ceux-ci soient efficaces ou non. Ces opérations de transfert et de sélection sont longues et délicates : la séquence étrangère est chargée sur une construction intermédiaire constituée par un vecteur plasmidique de transfert (ou de transplacement) et il faut, pour obtenir le vecteur d'expression, passer par la cotransfection et, donc, par la méthode de "marker rescue".

Il y a lieu de noter, par ailleurs, que dans un Article de SMITH, VLACK et SUMMERS intitulé "PHYSICAL ANALYSIS OF AUTOGRAPHA CALIFORNICA NUCLEAR POLYHEDROSIS VIRUS TRANSCRIPTS FOR POLYHEDRIN AND 10000-MOLECULAR-WEIGHT PROTEIN" paru dans JOURNAL OF VIROLOGY, Janvier 1983, pages 215-225, ces Auteurs décrivent les dimensions, la direction de transcription et l'emplacement des séquences d'ADN qui spécifient les extrêmités 5′ et 3′ de l'ARNm de la polyédrine de l′ AcMNPV (virus de la polyédrose nucléaire d'Autographa Californica), laquelle polyédrine est le polypeptide structural majeur de l'inclusion virale ; il est question, dans cet Article, d'une protéine de l'AcMNPV de bas poids moléculaire dans des gels SDS-PAGE, de l'ordre de 10 KDa, qui est produite dans des cellules-hôtes infectées, en quantités (élevées) comparables à celles de la polyédrine. Selon cet Article, la protéine P10 est, au même titre que la polyédrine, un gène de l'AcMNPV ; à 24 heures post-infection, on trouve dans les cellules infectées par l'AcMNPV une grande quantité de poly(A)⁺ARN qui s'hybride aux régions du génome où se trouvent localisés les gènes de la polyédrine et de la protéine P10 ; l'ARNm et les protéines de ces deux gènes qui s'accumulent dans les cellules infectées permettent de comprendre le contrôle de l'expression de ces gènes dans les cellules d'insectes dont l'étude est effectuée en utilisant comme modèles les séquences d'ADN responsables de l'expression préférée de la polyédrine et de la protéine P10 à un stade tardif de l'infection. La Demande de Brevet européen 0127 839 THE TEXAS A & M UNIVERSITY SYSTEMS analysée plus haut et dans lequelle SMITH & SUMMERS sont mentionnés en tant qu'Inventeurs, énonce et revendique au demeurant que le gène (ou fragment de gène) inséré dans le véhicule de clonage (tel qu'un plasmide), lui-même préalablement modifié par insertion d'un fragment d'ADN obtenu par clivage d'ADN de baculovirus et comprenant un gène de baculovirus (ou un fragment de gène), pour former le vecteur de baculovirus recombinant de transfert recherché, est un gène de polyédrine ou une fraction de celui-ci incluant le promoteur de polyédrine ou est un gène de protéine P10 incluant le promoteur de protéine P10. Les emplacements respectifs du gène de la polyédrine et du gène de la protéine P10 dans le génome de l'AcMNPV sont indiqués dans cette Demande de Brevet. Il est également mentionné dans cette dernière que c'est parce que le génome de l'AcMNPV n'a pas de sites de restriction uniques connus dans lesquels des gènes sélectionnés peuvent être effectivement introduits d'une manière site-spécifique, qu'il est nécessaire de construire des vecteurs plasmidiques chimériques (ou vecteurs de transfert) qui servent de véhicule intermédiaire pour le transfert des gènes.

La présente invention a en conséquence pour but de pourvoir à un vecteur d'expression qui puisse être obtenu sans avoir à passer par l'intermédiaire d'un vecteur de transfert.

Elle a également pour but de pourvoir à un vecteur d'expression non chargé, dans lequel il est possible de charger pratiquement n'importe quelle séquence. Elle a, de plus, pour but de pourvoir à un vecteur d'expression apte à être chargé d'une séquence étrangère directement in vitro par manipulation génétique.

La présente invention a pour objet un procédé de production d'un baculovirus modifié susceptible d'être utilisé en tant que vecteur d'expression de gènes exogènes, lequel procédé est caractérisé en ce qu'on insère dans le génome d'un baculovirus origine dépourvu de site de restriction pour au moins une enzyme donnée, un fragment d'ADN portant un site de restriction unique pour l'enzyme considérée, sous contrôle d'un promoteur tardif fort du gène de la polyédrine, ou de la protéine P10 - pour obtenir un virus modifié qui constitue un vecteur d'expression non chargé, prêt à recevoir au moins une séquence d'au moins un gène étranger que l'on désire faire exprimer.

Cette séquence étrangère peut être insérée directement, et sans passer par un vecteur de transfert.

Selon un mode de réalisation avantageux du procédé de production d'un baculovirus modifié, conforme à la présente invention, le baculovirus origine dépourvu de site de restriction par une enzyme donnée, mis en oeuvre, est un baculovirus naturellement dépourvu dudit site de restriction, tel, notamment, que le baculovirus de la polyédrose nucléaire de Spodoptera frugiperda (Sf).

Selon un autre mode de réalisation avantageux du procédé de production d'un baculovirus modifié, conforme à la présente invention, le baculovirus origine dépourvu de site de restriction pour une enzyme donnée, mis en oeuvre, est un baculovirus dont on a au préalable supprimé le ou les site(s) de restriction pour ladite enzyme, la suppression est réalisée par cotransfection de l'ADN du baculovirus origine considéré, avec un plasmide dans lequel a été cloné le fragment de l'ADN qui renferme le site de restriction qui a été coupé par une enzyme pour être linéarisé et auquel on a ligué un linker, puis que l'on a transformé, après ligation, en un plasmide dépourvu du site de restriction supprimé, ladite cotransfection donnant lieu à un virus recombinant dépourvu de ce site.

Selon un autre mode de réalisation avantageux du procédé de production d'un baculovirus modifié conforme à la présente invention, l'introduction d'un site de restriction pour une enzyme donnée, en aval du promoteur tardif fort du gène de la polyédrine ou/et du polypeptide P10 de baculovirus, est réalisée par cotransfection d'une culture cellulaire de cellules permissives au baculovirus, avec un plasmide approprié dans lequel a été cloné le fragment qui contient le site de restriction placé en aval du promoteur tardif fort, laquelle cotransfection aboutit à la formation de virus recombiné modifié, qui correspond au vecteur d'expression non chargé de séquence étrangère, prêt à recevoir au moins une séquence étrangère en vue de son expression sur cellules d'insectes.

Selon un autre mode de réalisation avantageux du procédé conforme à la présente invention, pour la construction d'un baculovirus modifié qui constitue un vecteur d'expression non chargé prêt à recevoir au moins deux fragments d'ADN étrangers que l'on désire faire exprimer, on installe sur un baculovirus convenablement choisi, au moins deux sites de restriction pour au moins deux enzymes identiques ou différentes.

Selon une disposition avantageuse de ce mode de réalisation, dans le cas où le baculovirus origine est dépourvu de deux sites de restriction pour deux enzymes identiques ou différentes, ces deux sites de restriction sont successivement introduits par cotransfection avec un premier plasmide dans lequel a été cloné le fragment qui contient le premier site de restriction que l'on souhaite installer sur le baculovirus, puis avec un second plasmide dans lequel a été cloné le fragment qui contient le second site de restriction que l'on cherche à installer sur le baculovirus.

Selon une autre disposition avantageuse de ce mode de réalisation, dans le cas où le baculovirus origine comporte des sites de restriction à supprimer sur son génome, ces sites sont successivement supprimés par cotransfection successivement avec des plasmides dépourvus de chacun des sites de restriction à supprimer. La présente invention a également pour objet un baculovirus modifié, susceptible d'être obtenu par le procédé conforme à l'Invention, comportant un site de restriction unique pour une enzyme donnée, installé en aval du promoteur tardif fort approprié du gène de la polyédrine ou de la P10.

Selon un mode de réalisation avantageux d'un tel baculovirus modifié, susceptible de constituer un vecteur d'expression non chargé, celui-ci est constitué par un baculovirus modifié comportant un site de restriction unique, installé à proximité du codon ATG du gène de la polyédrine ou de la P10, ou d'un des promoteurs tardifs forts de ces gènes.

Selon un autre mode de réalisation avantageux du baculovirus modifié conforme à la présente invention, il comporte au moins deux sites de restriction, chacun d'entre eux étant unique pour une enzyme donnée, installés respectivement à proximité du codon ATG du gène d'au moins une protéine associée au baculovirus et à proximité d'un autre promoteur tardif fort de ce virus.

La présente invention a en outre pour objet un vecteur d'expression de gènes, non chargé, constitué par un baculovirus modifié tel que défini dans ce qui précède.

La présente inention a, en outre, pour objet, un procédé de chargement d'un vecteur d'expression non chargé, en au moins une séquence étrangère, caractérisé en ce que ledit vecteur non chargé est constitué par un baculovirus modifié tel que défini ci-dessus, et en ce que au moins une séquence étrangère est introduite directement in vitro dans ledit vecteur d'expression, par manipulation génétique.

Selon une disposition avantageuse de ce mode de réalisation, pour l'introduction directe d'au moins une séquence étrangère, le génome viral est préalablement linéarisé par action d'une enzyme de restriction.

Selon un autre mode de réalisation avantageux dudit procédé de chargement, un fragment d'ADN étranger est introduit directement dans un premier site de restriction dudit vecteur d'expression non chargé, puis un second fragment d'ADN identique au premier ou différent de celui-ci, est introduit directement dans un second site de restriction dudit vecteur d'expression, les deux sites de restriction étant tous deux des sites de restriction uniques pour des enzymes différentes.

Selon encore un autre mode de réalisation avantageux dudit procédé de chargement, des fragments d'ADN étrangers identiques ou différents sont chargés dans des sites de restriction uniques, c'est-à-dire définis comme étant deux sites de restriction différents pour une enzyme identique, par digestion partielle dudit vecteur d'expression non chargé, pour ouvrir un site, dans lequel est alors introduit un premier fragment d'ADN, ledit vecteur étant soumis à une seconde digestion partielle pour ouvrir un second site unique identique au précédent, dans lequel est chargé un second fragment d'ADN.

Le vecteur d'expression conforme à l'invention peut être chargé de, pratiquement, n'importe quelle séquence attendu que lesdites séquences ne doivent pas comporter de sites de restriction particuliers.

C'est ainsi que sont susceptibles d'être exprimés par le vecteur d'expression conforme à l'invention, tous gènes codant pour des protéines d'origine procaryote et eucaryote, toutes séquences codant pour des gènes de biosynthèse, des antigènes protéiniques viraux et en particulier, à titre d'exemples non limitatifs, les gènes des acétylcholinestérases d'invertébrés et de vertébrés.

Les Publications citées précédemment et notamment les Demandes de Brevets européens citées plus haut, mentionnent que le gène de l'interféron β est exprimé d'autant plus efficacement s'il est inséré quelques nucléotides en amont du codon ATG initial du gène Pn, c'est-à-dire à proximité de ce codon ATG. Elles mentionnent également que les plasmides vecteurs recombinants obtenus contiennent la région du promoteur de polyédrine, avec, par exemple, un site BamHI unique en aval du site de départ transcriptionnel, c'est-à-dire situé environ 10 nucléotides en amont de l'emplacement du codon ATG du type sauvage, dans le vecteur d'insertion. En d'autres termes, l'Art antérieur propose la construction de vecteurs de clonage qui contiennent des sites de clonage uniques dans une position en aval d'un promoteur de baculovirus défini, qui sont flanqués de séquences homologues du génome viral, de façon à diriger l'insertion par recombinaison homologue.

Ces connaissances sont appliquées, conformément à la présente invention, à la production d'un vecteur d'expression non chargé.

La possibilité qu'offre la présente invention d'éviter le recours à un vecteur de transfert simplifie considérablement le procédé d'obtention d'un baculovirus recombiné chargé susceptible d'exprimer une protéine étrangère : en effet, alors que les procédés qui sont dans l'obligation de recourir à un vecteur de transfert requièrent plusieurs semaines pour produire un baculovirus recombiné chargé, le procédé conforme à la présente invention permet d'obtenir un baculovirus chargé, en quelques jours.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention dont ils ne constituent en aucune manière une limitation.

### EXEMPLE 1 : Suppression du site SmaI du génome du SlMNPV.

Pour construire le vecteur d'expression non chargé, c'est-à-dire le baculovirus modifié prêt à recevoir une séquence étrangère, on sélectionne un baculovirus dépourvu de site de restriction par une enzyme donnée : c'est le cas, exceptionnel, du baculovirus de la polyédrose nucléaire de Spodoptera frugiperda qui ne possède pas de site de restriction SmaI.

L'on peut aussi supprimer le ou les site(s) de restriction mal placé(s) sur le génome du baculovirus : notamment on supprime le site unique SmaI localisé dans le fragment Pst-I-G du baculovirus de la polyédrose nucléaire de Spodoptera littoralis, en procédant comme suit :

Le fragment Pst-I-G du SlMNPV cloné dans un plasmide de type pAT153 est coupé par SmaI, puis un linker KpnI est ligué au plasmide linéarisé. Les plasmides obtenus pas transformation après ligation et dépourvus de site SmaI sont cotransfectés avec le SlMNPV origine en vue d'obtenir des virus recombinants dépourvus de site SmaI. Dans la descendance des virus obtenus après cotransfection, on trie les virus recombinants. Ces virus auront, par rapport au virus origine, perdu un site SmaI, c'est-à-dire n'auront plus de site SmaI et auront par contre acquis un site KpnI et possèderont donc au total 7 + 1 = 8 sites KpnI. Pour sélectionner ces virus recombinants, on procède de la manière suivants : les polyèdres récoltés après la mort des chenilles transfectées sont source d'ADN qui sert pour une nouvelle transfection. Préalablement à cette nouvelle transfection, l'ADN est coupé par SmaI. Ce traitement à pour effet d'interdire la multiplication des virus d'origine car leur génome est ouvert, et de ne permettre que la multiplication des virus recombinants. Plusieurs cycles de multiplication des virus selon ce principe peuvent être nécessaires pour éliminer la descendance des virus d'origine ayant échappé à la coupure par SmaI.

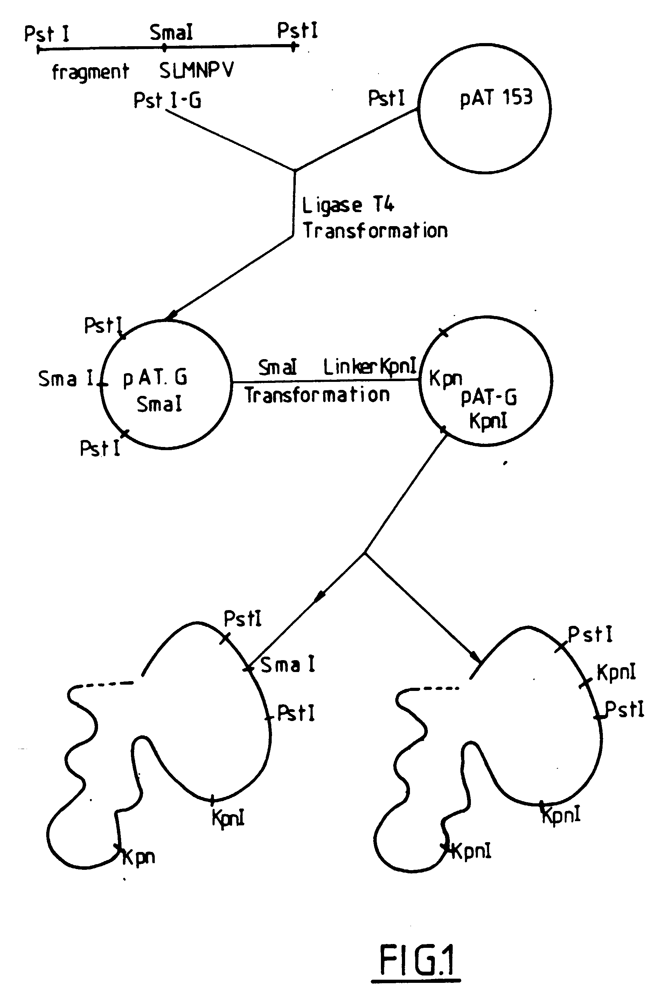

Ce processus de suppression d'un site de restriction SmaI mal placé est illustré dans la figure 1 annexée.

### EXEMPLE 2 : Installation d'un site SmaI en aval du promoteur de la polyédrine sur un baculovirus dépourvu de site de restriction SmaI :

Le site unique, qu'il s'agisse de SmaI ou de tout autre site, doit être placé dans la région de l'ATG qui est le codon initial du gène de la polyédrine (ou de P10). Quand on place ce site avant l'ATG, ceci permet l'expression du gène étranger sous le contrôle de son propre ATG (la position du site de chargement est dite en amont de l'ATG : c'est-à-dire du côté 5′ de l'ATG). Cette situation est la situation la plus fréquemment souhaitée. Quand on place le site après l'ATG (en aval de l'ATG, soit en 3′ par rapport à ce même ATG) la situation permet de produire des protéines dites fusionnées qui sous le contrôle de l'ATG de la polyédrine comportent en partie N-terminale des acides aminés correspondant à la séquence de la polyédrine et dans la partie C-terminale des acides aminés correspondant à la séquence étrangère fusionnée. La région d'installation du site s'étend à titre indicatif de -10n à +10n. Il est convenu que le A de l'ATG est numéroté +1, le T +2 et le G +3, les bases avant l'ATG sont numérotées avec le signe (-) ; la base qui précède A porte le numéro -1 et ainsi de suite.

Le choix de SmaI est conditionné par la relative rareté des sites SmaI chez les baculovirus. Le fait que l'ADN viral soit coupé à ce site de reconnaissance par l'endonucléase SmaI avec production d'extrêmités franches est certes un avantage dans la mesure où les extrêmités franches constituent une entrée universelle pour des séquences étrangères. Cet avantage n'est pas déterminant dans le choix car il est de pratique courante de rendre franches des extrêmités qui ne le sont pas au départ. Toute endonucléase de restriction capable de reconnaître un site unique de restriction positionné convenablement en aval d'un promoteur, permet l'introduction de séquences étrangères, en vue de l'expression de gènes chargés, au prix de modifications parfois mineures des extrêmités de celles-ci.

Conformément à l'invention, il faut, sur un virus dépourvu de site de restriction par SmaI par exemple, soit NPV de Spodoptera frugiperda, soit NPV de Spodoptera littoralis traité conformément à l'Exemple 1, installer un site SmaI (qui devient pour le génome viral un site unique) en aval du promoteur du gène de la polyédrine (ou de tout autre promoteur tardif fort comme celui de la protéine P10). L'intallation du site SmaI au meilleur endroit suppose l'établissement préalable de la séquence du gène de la polyédrine (notamment de la région promotrice).

Pour installer le site SmaI il faut disposer d'un site de restriction unique qui permettre d'ouvrir le génome dans une partie restreinte du gène de la polyédrine. Les sites uniques chez le virus entier sont rares. La probabilité de trouver un site unique augmente lorsqu'on réduit la taille de la séquence.

Cette introduction peut se faire par plusieurs méthodes. Parmi celles-ci, on peut citer la mutation dirigée avec création de site unique, suivie de l'introduction dans ces sites de linker SmaI.

Une autre méthode consiste à procéder à des délétions induites par Bal31 à la suite de la coupure de la séquence virale au niveau du site SacI. Comme il existe un second site SacI dans le polylinker du plasmide, il est nécessaire, préalablement à la digestion par Ba131, de faire sauter du polylinker, ce site SacI.

Le polylinker a, initialement, la composition suivante : EcoRI ; Sac1 ; Kpn1 ; Sma1 ; BamH1 ; Xba1 ;
Sal1 ; PstI ; SphI ; HindIII

Après le clonage du fragment HindIII dans le pUC19, on procède à une délétion dirigée avec le système ExoIII-Mung bean. Le site de blocage est le site PstI contenu en 5′ du polylinker et le site de digestion un site HpaI situé entre le polylinker et le site XbaI du fragment HindIII-K. Cette délétion fait disparaître un segment compris entre le site PstI et le site HpaI d'une part et un segment situé entre ce même site HpaI et le site XbaI viral.

Le plasmide délété est préparé pour un séquençage direct (donc simplement pour des raisons de commodité) des plasmides délétés ultérieurement autour du site XbaI de la séquence virale. A ce stade le polylinker a la composition suivante :

EcoRI;SacI;KpnI;SmaI;BamHI;XbaI;SalI et le premier site significatif viral en 3′ est XbaI.

On élimine alors le site SacI du polylinker par une double digestion EcoRI-BamHI qui fait subsister du polylinker uniquement les sites XbaI et SalI. De cette manière on peut à la fois procéder à une délétion autour du site viral SacI et installer, à la place de cette délétion, un linker SmaI qui sera site unique dans ce plasmide. La position du site SmaI doit être de -30 à +10 puisque XbaI est en position -40 par rapport au A de l'ATG.

Les plasmides p13.21XSS sont cotransfectés sur culture cellulaire de S.littoralis et les virus recombinés qui correspondent aux vecteurs d'expression non chargés de séquence étrangère sont repérés par le phénotype "P moins". Ce phénotype est celui des virus responsables de foyers infectieux par la méthode des plages dans lesquelles les polyèdres ne peuvent être mis en évidence.

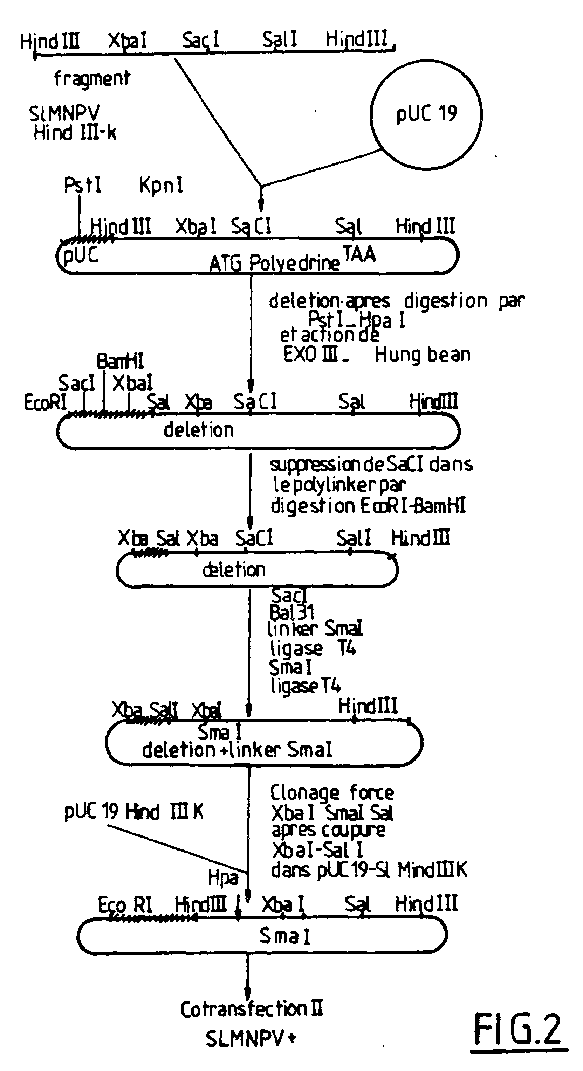

Ce processus d'installation d'un site de restriction SmaI est illustré dans la figure 2 annexée.

### EXEMPLE 3 : Chargement de séquence étrangère en amont immédiat de l'ATG.

Cette étape de la construction du vecteur d'expression présuppose la possession du baculovirus modifié conformément à l'invention, c'est-à-dire du virus possédant un site unique situé par exemple à -4 (la référence de numérotation étant toujours +1 pour le A de l'ATG initial du gène de la polyédrine). Ledit virus modifié est en fait amputé des bases qui s'étendent de -3 à environ +280 puisque le site SacI à partir duquel Ba131 digère, est situé à +140. L'obtention du vecteur suppose la préparation d'une séquence étrangère apte à être exprimée.

### Séquence chargée : Séquence de l'acétylcholinestérase de Drosophila melanogaster.

La séquence à exprimer est issue de la forme réplicative du plasmide pEMBL-Ache de l'acétylcholinestérase de D.melanogaster (plasmide pE6 obtenu par Fournier et al. INRA, Antibes). La séquence publiée à l'origine par Hall et Spierer [EMBO. J : (1986), page 2952] est longue de 3481 bases. Le segment NruI-SacI qui s'étend de +869 à +3181 peut être utilement souscloné dans un pUC19 en SmaI-SacI. NruI coupe le gène de l'acétylcholinestérase 127 bases en amont de l'ATG de ce gène. Un site unique AatII à +921 permet la récupération du fragment AatII-SacI qui comporte la totalité du gène Ache (soit une séquence leader de 72 bases, un ORF de 1947 bases et une séquence en 3′ de 241 bases comprenant un signal de polyadénylation à +3050).

Afin d'éviter la présence de la séquence codant pour la queue d'ancrage et de faciliter la purification de l'acétylcholinestérase, l'emploi du segment AatII-XmnI (+921 - +2856) qui donne une protéine tronquée de 28 acides aminés sur 649 acides aminés de drosophile en aval du gène Ache, est très favorable. Le résultat de ce montage conduit à une protéine fusionnée en 3′. La nature de la protéine fusionnée est fonction des résidus obtenus par la délétion induite par Bal31. Après la séquence AGTGGG formée du dernier codon de l'Ache et du codon GGG qui est un demi dite SmaI suit, soit les bases du gène de la polyédrine dans le bon cadre de lecture auquel cas la protéine fusionnée comporte environ 120 acides aminés de polyédrine, soit des bases dans un mauvais cadre de lecture et il apparaît des codons stop après une trentaine de codons sens.

Les extrêmités du fragment d'ADN double-brin à insérer dans le virus modifié au niveau du site SmaI sont rendues franches si nécessaire par l'action de Mung-bean pour les sites SacI et AatII.

Après transfection de cellules de Spodoptera littoralis, l'activité acétylcholinestérasique est mesurée à partir d'une série de surnageants et/ou de broyats provenant de X cellules isolées à 48 heures post-infection et est maintenue 48 heures supplémentaires pour la production d'acétylcholinestérase. Les lots associés à une activité enzymatique élevée donnent lieu à un clonage par dilution limite. La présence de clones positifs est recherchée par hybridation avec une sonde pUC-Ache.

### EXEMPLE 4 : Chargement en aval de l'ATG (cas des protéines fusionnées). Expression du gène de la bétagalactosidase.

Cet exemple, comme le précédent, suppose l'existence du baculovirus modifié. Dans ce cas la délétion produite par Ba131 à partir du site SacI respecte l'ATG.. Le fragment SmaI-SalI du plasmide pMC1871 de Casadaban (plasmide distribué par Pharmacia) est inséré dans le site SmaI du virus transformé après modification de l'extrêmité SalI par Mung bean.

Cette construction présente la structure : < ----promoteur de la polyédrine------ATG TAT--<---GGG GAT CCC GTC----LacZ----AAA TAA TAA TAA CCG GGC AGG GGG GAT CCG->--- extrêmité 3′ de l'ORF de la polyédrine.---->

Les virus recombinés ayant cette structure sont reconnus en culture cellulaire par la coloration bleue avec X-gal.

## Revendications

1. Procédé de production d'un baculovirus modifié susceptible d'être utilisé en tant que vecteur d'expression de gènes exogènes, lequel procédé est caractérisé en ce qu'on insère dans le génome d'un baculovirus origine dépourvu de site de restriction pour au moins une enzyme donnée, un fragment dADN portant un site de restriction unique pour l'enzyme considérée, sous contrôle d'un promoteur tardif fort du gène de la polyédrine, ou de la protéine P10 - pour obtenir un virus modifié qui constitue un vecteur d'expression non chargé, prêt à recevoir directement et sans passer par l'intermédiaire d'un vecteur de transfert, au moins une séquence d'au moins un gène étranger que l'on désire faire exprimer.

2. Procédé selon la revendication 1, caractérisé en ce que le baculovirus origine dépourvu de site de restriction pour une enzyme donnée, mis en oeuvre, est un baculovirus naturellement dépourvu dudit site de restriction.

3. Procédé selon la revendication 2, caractérisé en ce que ledit baculovirus origine est le baculovirus de la polyédrose nucléaire de *Spodoptera frugiperda* (Sf), et ladite enzyme de restriction est SmaI.

4. Procédé selon la revendication 1, caractérisé en ce que le baculovirus origine dépourvu de site de restriction pour une enzyme donnée, mis en oeuvre, est un baculovirus dont on a au préalable supprimé le ou les site(s) de restriction pour ladite enzyme.

5. Procédé selon la revendication 4, caractérisé en ce que le baculovirus origine est le baculovirus de la polyédrose nucléaire de *Spodoptera littoralis* et ladite enzyme est SmaI.

6. Procédé selon l'une quelconque des revendications 1 à 5, caractérisé en ce que l'introduction d'un site de restriction unique pour une enzyme donnée, en aval du promoteur tardif fort du gène de la polyédrine ou du polypeptide P10 de baculovirus, est réalisée par cotransfection d'une culture cellulaire de cellules permissives au baculovirus, avec un plasmide dans lequel a été cloné le fragment qui contient le site de restriction placé en aval du promoteur tardif fort, laquelle cotransfection aboutit à la formation de virus recombiné modifié, qui correspond au vecteur d'expression non chargé de séquence étrangère, prêt à recevoir une séquence étrangère en vue de son expression sur cellules d'insectes.

7. Procédé selon l'une quelconque des revendications 1 à 6, caractérisé en ce que, pour la construction d'un baculovirus modifié qui constitue un vecteur d'expression non chargé prêt à recevoir au moins deux fragments d'ADN étrangers que l'on désire faire exprimer, on installe sur un baculovirus convenablement choisi, au moins deux sites de restriction pour au moins deux enzymes identiques ou différentes, chacun desdits sites étant unique pour l'enzyme correspondante.

8. Procédé selon la revendication 7, caractérisé en ce que les deux sites de restriction sont successivement introduits par cotransfection avec un premier plasmide dans lequel a été cloné le fragment qui contient le premier site de restriction que l'on souhaite installer sur le baculovirus, puis avec un second plasmide dans lequel a été cloné le fragment qui contient le second site de restriction que l'on cherche à installer sur le baculovirus.

9. Baculovirus modifié caractérisé en ce qu'il est susceptible d'être obtenu par un procédé selon l'une quelconque des revendications 1 à 8 et comporte au moins un site de restriction unique pour une enzyme donnée installé en aval du promoteur tardif fort du gène de la polyédrine ou de la P10, lequel baculovirus est apte à constituer un vecteur d'expression non chargé.

10. Baculovirus modifié, selon la revendication 9, caractérisé en ce qu'il est constitué par un baculovirus modifié comportant un site de restriction installé à proximité du codon ATG du gène de la polyédrine ou de la P10.

11. Baculovirus modifié, selon la revendication 9, caractérisé en ce que le baculovirus origine est le baculovirus de la polyédrose nucléaire de *Spodoptera frugiperda* ou de *Spodoptera littoralis,* et ce que le site unique est un site SmaI.

12. Baculovirus modifié selon la revendication 9, caractérisé en ce qu'il comporte au moins deux sites de restriction, chacun d'entre eux étant unique pour une enzyme donnée, installés respectivement à proximité du codon ATG du gène et à proximité d'un promoteur tardif fort dudit gène.

13. Vecteur d'expression de gènes, non chargé, caractérisé en ce qu'il est constitué par un baculovirus modifié selon l'une quelconque des revendications 9 à 12.

14. Procédé de chargement d'un vecteur d'expression non chargé, en au moins une séquence étrangère, caractérisé en ce que ladite séquence étrangère est introduite directement, sans recourir à un vecteur de transfert, au site de restriction unique présent dans le génome viral modifié qui constitue le vecteur d'expression initialement non chargé selon la revendication 13.

15. Procédé de chargement selon la revendication 14, caractérisé en ce que pour l'introduction directe d'au moins une séquence étrangère au site unique pour une enzyme donnée, le génome viral est préalablement linéarisé par action de ladite enzyme de restriction.

16. Procédé de chargement selon l'une quelconque des revendications 14 ou 15, caractérisé en ce qu'un fragment d'ADN étranger est introduit directement dans un premier site de restriction dudit vecteur d'expression non chargé, puis un second fragment d'ADN identique au premier ou différent de celui-ci est introduit directement dans un second site de restriction dudit vecteur d'expression, les deux sites de restriction étant tous deux des sites de restriction uniques pour des enzymes différentes.

17. Procédé de chargement selon l'une quelconque des revendications 14 ou 15, caractérisé en ce que des fragments d'ADN étrangers identiques ou différents sont chargés dans des sites de restriction uniques, c'est-à-dire définis comme étant deux sites de restriction pour une enzyme identique, situés à des emplacements différents, par digestion partielle dudit vecteur d'expression non chargé, pour ouvrir un site, dans lequel est alors introduit un premier fragment d'ADN, ledit vecteur étant soumis à une seconde digestion partielle pour ouvrir un second site unique identique au précédent dans lequel est chargé un second fragment d'ADN.

## Patentansprüche

1. Verfahren zur Produktion eines modifizierten Baculovirus, das als Expressionsvektor für exogene Gene verwendet werden kann, dadurch **gekennzeichnet**, daß man in das aus einem Baculovirus stammende Genom ohne Restriktionsspaltstelle für mindestens ein gegebenes Enzym ein DNA-Fragment, das eine für das in Betracht gezogene Enzym einzigartige Restriktionsspaltstelle trägt, unter der Kontrolle eines starken späten Promotors für das Polyhedrin-Gen oder das Protein-P10-Gen insertiert, um ein modifiziertes Virus zu erhalten, das einen nicht-beladenen Expressionsvektor, der direkt und ohne den Weg über einen Transfervektor als Zwischenprodukt mindestens eine Sequenz mindestens eines Fremdgens, das exprimiert werden soll, aufnehmen kann, darstellt.

2. Verfahren nach Anspruch 1, dadurch **gekennzeichnet**, daß das eingesetzte ursprüngliche Baculovirus ohne Restriktionsspaltstelle für ein gegebenes Enzym ein Baculovirus ist, das natürlicherweise ohne die Restriktionsspaltstelle ist.

3. Verfahren nach Anspruch 2, dadurch **gekennzeichnet**, daß das ursprüngliche Baculovirus das Baculovirus der nuklearen Polyhedrose von *Spodoptera frugiperda* (Sf) ist und das Restriktionsenzym SmaI ist.

4. Verfahren nach Anspruch 1, dadurch **gekennzeichnet**, daß das eingesetzte ursprüngliche Baculovirus ohne Restriktionsspaltstelle für ein gegebenes Enzym ein Baculovirus ist, bei dem zuvor die Restriktionsspaltstelle(n) für das Enzym suprimiert wurde(n).

5. Verfahren nach Anspruch 4, dadurch **gekennzeichnet**, daß das ursprüngliche Baculovirus das Baculovirus der nuklearen Polyhedrose von *Spodoptera littoralis* ist und das Enzym SmaI ist.

6. Verfahren nach einem der Ansprüche 1 bis 5, dadurch **gekennzeichnet**, daß die Einschleusung einer einzigartigen Restriktionsspaltstelle für ein gegebenes Enzym stromabwärts des starken späten Promotors für das Polyhedrin-Gen oder das Polypeptid-P10-Gen des Baculovirus durch Co-Transfektion einer Zellkultur von für das Baculovirus permissiven Zellen mit einem Plasmid durchgeführt wird, in das das Fragment cloniert wurde, das die Restriktionsspaltstelle stromabwärts des starken späten Promotors enthält, wobei die Co-Transfektion zur Bildung von modifizierten rekombinanten Viren führt, die dem mit der Fremdsequenz nicht-beladenen Expressionsvektor, der eine Fremdsequenz im Hinblick auf deren Expression in Insektenzellen aufnehmen kann, entspricht.

7. Verfahren nach einem der Ansprüche 1 bis 6, dadurch **gekennzeichnet**, daß zur Konstruktion eines modifizierten Baculovirus, das einen nicht-beladenen Expressionsvektor, der mindestens zwei zu exprimierende Fremd-DNA-Fragmente aufnehmen kann, man in ein geeigneter Weise ausgewähltes Baculovirus mindestens zwei Restriktionsspaltstellen für mindestens zwei identische oder unterschiedliche Enzyme einführt, wobei jede der Spaltstellen für das entsprechende Enzym einzigartig ist.

8. Verfahren nach Anspruch 7, dadurch **gekennzeichnet**, daß die zwei Restriktionsspaltstellen successive durch Co-Transfektion mit einem ersten Plasmid eingeschleust werden, in das das Fragment cloniert wurde, das die erste Restriktionsspaltstelle, die in das Baculovirus eingeführt werden soll, enthält, anschließend mit einem zweiten Plasmid eingeschleust werden, in das das Fragment cloniert wurde, das die zweite Restriktionsspaltstelle, die in das Baculovirus eingeführt werden soll, enthält.

9. Modifiziertes Baculovirus, dadurch **gekennzeichnet**, daß es durch ein Verfahren nach einem der Ansprüche 1 bis 8 erhalten werden kann und mindestens eine einzigartige Restriktionsspaltstelle für ein gegebenes Enzym stromaufwärts des starken späten Promotors des Polyhedrin-Gens oder des P10-Gens enthält, wobei das Baculovirus einen nicht-beladenen Expressionsvektor darstellen kann.

10. Modifiziertes Baculovirus nach Anspruch 9, dadurch **gekennzeichnet**, daß es aus einem modifizierten Baculovirus, umfassend eine in der Nähe des ATG-Codons des Polyhedrin- oder P10-Gens eingeführte Restriktionsspaltstelle umfaßt.

11. Modifiziertes Baculovirus nach Anspruch 9, dadurch **gekennzeichnet**, daß das ursprüngliche Baculovirus das Baculovirus der nuklearen Polyhedrose von *Spodoptera frugiperda* oder *Spodoptera littoralis* ist, und das die einzigartige Restriktionsspaltstelle eine SmaI-Spaltstelle ist.

12. Modifiziertes Baculovirus nach Anspruch 9, dadurch **gekennzeichnet**, daß es mindestens zwei Restriktionsspaltstellen enthält, wobei jede von den beiden für ein gegebenes Enzym einzigartig ist, die jeweils in der Nähe des ATG-Codons und des starken späten Promotors des Gens installiert sind.

13. Nicht-beladener Expressionsvektor für Gene, dadurch **gekennzeichnet**, daß er aus einem modifizierten Baculovirus nach einem der Ansprüche 9 bis 12 besteht.

14. Verfahren zur Beladung eines nicht-beladenen Expressionsvektors mit mindestens einer Fremdsequenz, dadurch **gekennzeichnet**, daß die Fremdsequenz direkt ohne Umweg über einen Transfervektor an der einzigartigen in dem viralen modifizierten Genom vorhandenen Restriktionsspaltstelle, das den anfänglich nicht-beladenen Expressionsvektor nach Anspruch 13 darstellt, eingeschleust wird.

15. Verfahren zur Beladung nach Anspruch 14, dadurch **gekennzeichnet**, daß zur direkten Einschleusung mindestens einer Fremdsequenz an der für ein gegebenes Enzym einzigartigen Restriktionsspaltstelle das virale Genom zuvor durch Wirkung des Restriktionsenzyms linearisiert wird.

16. Verfahren zur Beladung nach einem der Ansprüche 14 oder 15, dadurch **gekennzeichnet**, daß ein Fremd-DNA-Fragment direkt in eine erste Restriktionsspaltstelle des nicht-beladenen Expressionsvektors eingeschleust wird, dann ein dem ersten Fragment identisches zweites DNA-Fragment oder ein davon unterschiedliches zweites DNA-Fragment direkt in eine zweite Restriktionsspaltstelle des Expressionsvektors eingeschleust wird, wobei die zwei Restriktionsspaltstellen beide für unterschiedliche Enzyme einzigartige Restriktionsspaltstellen sind.

17. Verfahren zur Beladung nach einem der Ansprüche 14 oder 15, dadurch **gekennzeichnet**, daß die identischen oder unterschiedlichen Fremd-DNA-Fragmente in einzigartige Restriktionsspaltstellen, d.h., die so definiert sind, daß sie zwei Restriktionsspaltstellen für ein identisches Enzym sind, an unterschiedlichen Orten angeordnet sind, durch Teilspaltung des nicht-beladenen Expressionsvektors beladen werden, um eine Spaltstelle zu öffnen, in die dann ein erstes DNA-Fragment eingeschleust wird, wobei der Vektor einer zweiten Teilspaltung unterworfen wird, um eine zweite der vorhergehenden Spaltstelle identische einzigartige Spaltstelle zu öffnen, in die ein zweites DNA-Fragment eingeschleust wird.

## Claims

1. Process for the production of a modified baculovirus which can be used as an expression vector for exogenic genes, which process is characterised in that there is inserted in the genome of a starting baculovirus devoid of a restriction site for at least one given enzyme, a DNA fragment carrying a restriction site unique for the enzyme concerned, under the control of a strong late promoter of the polyhedrin or protein P10 gene - to obtain a modified virus which constitutes a non-charged expression vector ready to receive directly and without passing via the intermediary of a transfer vector at least one sequence of at least one foreign gene which it is desired to express.

2. Process according to Claim 1,
characterised in that the starting baculovirus used, which is devoid of a restriction site for a given enzyme, is a baculovirus that is naturally devoid of the said restriction site.

3. Process according to Claim 2,
characterised in that the starting baculovirus is the baculovirus of the nuclear polyhedrose of Spodoptera frugiperda (Sf) and the restriction enzyme is SmaI.

4. Process according to Claim 1,
characterised in that the starting baculovirus used, which is devoid of a restriction site for a given enzyme, is a baculovirus of which the restriction site(s) for the said enzyme has (have) been suppressed beforehand.

5. Process according to Claim 4,
characterised in that the starting baculovirus is the baculovirus of the nuclear polyhedrose of Spodoptera littoralis and the enzyme is SmaI.

6. Process according to any one of Claims 1 to 5, characterised in that the introduction of a restriction site unique for a given enzyme, downstream of the strong late promoter of the polyhedrin or polypeptide P10 gene of baculovirus, is carried out by cotransfection of a cell culture of cells permissive to the baculovirus, with a plasmid in which the fragment containing the restriction site placed downstream of the strong late promoter has been cloned, which cotransfection results in the formation of modified recombinant virus, which corresponds to the expression vector not charged with a foreign sequence, ready to receive a foreign sequence with a view to its expression on insect cells.

7. Process according to any one of Claims 1 to 6, characterised in that, for the construction of a modified baculovirus which constitutes a non-charged expression vector ready to receive at least two foreign DNA fragments which it is desired to express, at least two restriction sites for at least two identical or different enzymes are installed on a suitably selected baculovirus, each of the said sites being unique for the corresponding enzyme.

8. Process according to Claim 7,
characterised in that the two restriction sites are introduced in succession by cotransfection with a first plasmid in which the fragment containing the first restriction site which it is desired to install on the baculovirus has been cloned, then with a second plasmid in which the fragment containing the second restriction site which it is desired to install on the baculovirus has been cloned.

9. Modified baculovirus, characterised in that it can be obtained by a process according to any one of Claims 1 to 8 and comprises at least one restriction site that is unique for a given enzyme and that is installed downstream of the strong late promoter of the polyhedrin or P10 gene, which baculovirus is suitable as a non-charged expression vector.

10. Modified baculovirus according to Claim 9, characterised in that it is constituted by a modified baculovirus comprising a restriction site installed in the vicinity of the ATG codon of the polyhedrin or P10 gene.

11. Modified baculovirus according to Claim 9, characterised in that the starting baculovirus is the baculovirus of the nuclear polyhedrose of Spodoptera frugiperda or Spodoptera littoralis, and in that the unique site is an SmaI site.

12. Modified baculovirus according to Claim 9, characterised in that it comprises at least two restriction sites, each of which is unique for a given enzyme and which are installed respectively in the vicinity of the ATG codon of the gene and in the vicinity of a strong late promoter of the said gene.

13. Non-charged gene expression vector, characterised in that it is constituted by a modified baculovirus according to any one of Claims 9 to 12.

14. Process for charging a non-charged expression vector with at least one foreign sequence, characterised in that the said foreign sequence is introduced directly, without using a transfer vector, at the unique restriction site present in the modified viral genome which constitutes the initially non-charged expression vector according to Claim 13.

15. Charging process according to Claim 14, characterised in that, for the direct introduction of at least one foreign sequence at the site unique for a given enzyme, the viral genome is previously linearised by the action of the said restriction enzyme.

16. Charging process according to either Claim 14 or Claim 15, characterised in that a foreign DNA fragment is introduced directly into a first restriction site of the said non-charged expression vector, then a second DNA fragment identical to the first or different therefrom is introduced directly into a second restriction site of the said expression vector, the two restriction sites both being restriction sites unique for different enzymes.

17. Charging process according to either Claim 14 or Claim 15, characterised in that identical or different foreign DNA fragments are charged into unique restriction sites, that is to say, restriction sites defined as being two restriction sites for an identical enzyme, located at different places, by partial digestion of the said non-charged expression vector in order to open a site into which a first DNA fragment is then introduced, the said vector being subjected to a second partial digestion in order to open a second unique site identical to the previous one into which a second DNA fragment is charged.
